# EUROPÄISCHE PATENTSCHRIFT

(11) **EP 0 897 714 B1**
(45) Veröffentlichungstag und Bekanntmachung des Hinweises auf die Patenterteilung: **21.01.2004**
(21) Anmeldenummer: 98114962.8
(22) Anmeldetag: 08.08.1998
(51) Int. Cl.: A61K 7/20

(54) **Zweikomponenten-Mundspülmittel**
Two-component mouthwash composition
Bain de bouche à deux composants

(30) Priorität: 18.08.1997 DE 19735779
(43) Veröffentlichungstag der Anmeldung: 24.02.1999
(73) Patentinhaber: Henkel Kommanditgesellschaft auf Aktien, 40589 Düsseldorf-Holthausen (DE)
(72) Erfinder: Pastura, Amerigo, Dr. Dipl.-Chem., 58453 Wiiten (DE); Walther-Stangrecki, Claudia, Chemielab., 40764 Langenfeld (DE); Casa, Paco, 08017 Barcelona (ES); Pujol, Miracle, 08329 Reia (ES)

(56) Entgegenhaltungen:
- EP-A2- 0 202 359
- WO-A1-97/02805
- US-A- 5 392 947

## Beschreibung

Die Erfindung betrifft ein Mundspülmittel, das aus zwei getrennt verpackten Komponenten unmittelbar vor der Anwendung gemischt wird und dessen eine Komponente Wasserstoffperoxid enthält.

Die Anwendung von Peroxiden als Komponente von Mund- und Zahnpflegemitteln ist schon sehr lange bekannt, zum einen da Peroxyverbindungen eine antimikrobielle Wirkung gegen viele schädlichen Keime der Mundhöhle entfalten und auf diese Weise zur Behandlung von Gingivitis und Periodontitis sowie zur Bekämpfung der Zahnplaque beitragen. Zum anderen bewirken Peroxide aufgrund ihrer bleichenden und oxidierenden Wirkung eine Aufhellung dunkel verfärbter Zähne und tragen damit ganz erheblich zum Reinigungserfolg von Zahnpasten und Mundwässem bei.

Ein Nachteil von peroxidhaltigen Zubereitungen ist die mangelnde Stabilität von Peroxiden in wäßriger Lösung, die zu einem Verlust an aktivem Peroxidsauerstoff im Verlauf längerer Lagerung, insbesondere bei höheren Umgebungstemperaturen führen kann. Dieses Problem ist besonders gravierend, wenn in der Zubereitung oxidierbare organische Komponenten oder solche Komponenten enthalten sind, die einen neutralen oder schwach basischen pH-Wert verursachen oder sonst eine Aktivierung des Peroxidsauerstoffs bedingen.

Ein weiteres bekanntes Problem besteht darin, daß Peroxide in ihrer Oxidationswirkung ziemlich unspezifisch sind und daher bei höherer Dosierung, wie sie für die Bleichung verfärbter Zähne wünschenswert wäre, zu Verätzungen der Mundschleimhaut führen können. Aus diesem Grunde sind in manchen Ländern Konzentrations-Obergrenzen für den Einsatz von z.B. Wasserstoffperoxid in mundhygienischen Zubereitungen gesetzlich festgelegt.

Es hat daher nicht an Versuchen gefehlt, peroxidhaltige Mund- und Zahnpflegemittel gegen den Peroxidzerfall zu stabilisieren, um auch bei den gewünschten niedrigen Einsatzkonzentrationen über längere Zeit eine befriedigende Wirkung zu erzielen. Dabei hat man auch schon den Vorschlag gemacht, das Peroxid in einer separaten Zubereitung bereitzustellen, die erst kurz vor der Anwendung mit einer zweiten Zubereitung vermischt wird, welche die übrigen Komponenten des Mundpflegemittels enthält. Diese zweite Zubereitung kann dann z.B. schwach basische oder andere das Peroxid destabilisierende Komponenten enthalten - oder sie kann Aktivatoren bzw. Katalysatoren enthalten, die das Peroxid kurz vor der Anwendung in der Wirkung verstärken.

Aus WO 97/02805 A1 ist z.B. ein Zweikomponenten-Mundwasser bekannt, dessen erste Komponente das Wasserstoffperoxid und dessen zweite Komponente einen Mangan-Komplex als Peroxidaktivator enthält, um die Bleichwirkung des Mundwassers zu erhöhen. Aus US 5,392,947 A ist ein weiteres Zweikomponenten-Mundwasser bekannt, dessen erste Komponente das Peroxid und eine Säure enthält und dessen zweite Komponente Natriumbicarbonat enthält, so daß bei Vereinigung der Komponenten durch Freisetzung von Kohlendioxid ein perlendes oder sprudelndes Mundspülmittel entsteht.

Es wurde nun überraschend festgestellt, daß durch Einhaltung definierter pH-Wert-Bereiche für die beiden Komponenten eines Zweiphasen-Mundspülmittels und der daraus durch Mischen vor der Anwendung gebildeten, gebrauchsfertigen Spüllösung unter Verwendung geeigneter Säure- und Pufferkomponenten die Bleich- und Reinigungsleistung solcher Zubereitungen erheblich gesteigert werden kann.

Gegenstand der Erfindung ist daher ein Mundspülmittel, bestehend aus zwei getrennt verpackten Komponenten, die in einer Verkaufseinheit zusammengefaßt sind und vor der Anwendung miteinander gemischt werden, dadurch gekennzeichnet, daß
- die erste Komponente eine wäßrige Lösung von water 0,5 Gew.-% Wasserstoffperoxid und eine Säure zur Einstellung eines pH-Wertes unter 4,5 und
- die zweite Komponente eine wäßrige oder wäßrig-alkoholische Lösung mit einem Gehalt von einem oder mehreren Stoffen aus der Gruppe der Tenside, Geschmacksstoffe oder mundhygienische Wirkstoffe und 1-10 Gew.-% eines Puffersalzes einer nichtflüchtigen Säure enthält und einen pH-Wert oberhalb 8 aufweist und
- die Mischung der beiden Komponenten eine Lösung mit einem Wasserstoffperoxid-Gehalt von mindestens 0,005 Gew.-% und mit einem pH-Wert von 7 oder höher ergibt.

Der Wasserstoffperoxid-Gehalt der ersten Komponente beträgt bevorzugt 0,1-6 Gew.-% und das Mischungsverhältnis (in Volumenteilen) der beiden Komponenten liegt bevorzugt bei 1 : 1. Dadurch wird es z.B. möglich, einen Zweikomponenten-Spendebehälter zu benutzen, der beim Ausgießen beide Komponenten gleichzeitig und in gleicher Menge freigibt. Es erscheint aber auch technisch möglich, die Wasserstoffperoxid-Konzentration der ersten Komponente zu erhöhen und z.B. einen Zweikomponenten-Spendebehälter mit unterschiedlich großen Kammern zu benützen, die durch entsprechende Dimensionierung der Ausgußöffnungen die Komponenten in einem Mischungsverhältnis abgeben, das dem Verhältnis der Kammervolumina entspricht.

Als Säuren zur Einstellung des pH-Werts der ersten Komponente können alle anorganischen und organischen Säuren verwendet werden, wobei den physiologisch besser verträglichen Produkten wie z.B. der Phosphorsäure, oder den organischen Hydroxycarbonsäuren, z.B. der Milchsäure, Zitronensäure, Weinsäure, Äpfelsäure oder der Ascorbinsäure der Vorzug gegeben wird. Besonders bevorzugt eignet sich eine komplexbildende Hydroxycarbonsäure wie die Zitronensäure.

Die Mengen an Säure, die zur Einstellung eines pH-Wertes von weniger als 4,5 erforderlich sind, liegen z.B. bei Abwesenheit von Puffern bei 0,001 Gew.-% Zitronensäure-monohydrat. Bevorzugt wird aber auch der Komponente A ein Puffersalz, z.B. Natriumbenzoat, zugegeben. In diesem Falle sind entsprechend höhere Mengen an Säure zur Einstellung eines pH-Wertes von weniger als 4,5 erforderlich.

Die zweite Komponente enthält als wichtigste Komponente ein Puffersalz einer nichtflüchtigen Säure. Puffersalze im Sinne der Erfindung sind Salze von starken Basen und schwachen Säuren, wobei die Salze der Kohlensäure (Bicarbonate) oder von niedrigsiedenden organischen Säuren (Formiate, Acetate) weniger geeignet sind.

Geeignete Puffersalze sind z.B. die Alkalisalze von Milchsäure, Zitronensäure, Weinsäure, Äpfelsäure (sog. Fruchtsäuren), Malonsäure, Bernsteinsäure, Benzoesäure, p-Hydroxybenzoesäure, Salicylsäure oder Ascorbinsäure. Bevorzugt geeignet sind die Alkalisalze von komplexbildenden Säuren aus der Gruppe der Hydroxy- oder Aminopolycarbonsäuren, der Organophosphonsäuren, z.B. der Hydroxy- oder Aminoalkandiphosphonsäuren, der Phosphonoalkanpolycarbonsäuren oder der kondensierten Oligophosphorsäuren. Beispiele solcher Salze sind z.B. die Citrate, Tartrate, Nitrilotriacetate, Ethylendiamintetraacetate, 1-Hy-droxyethan-1,1-diphosphonate, Azacycloalkandiphosphonate, Pyro- und Tripoly- und Trimetaphosphate. Bevorzugt ist als Puffersalz Trinatriumcitrat in einer Menge von 3 - 6 Gew.-% in der zweiten Komponente (B) enthalten.

Die Puffersalze liegen erfindungsgemäß in einer Menge von 1-10 Gew.-% in der zweiten Komponente des Mundspülmittels vor, so daß in der gebrauchsfertigen Mundspüllösung eine Konzentration von 0,5 - 5 Gew.-% des Puffersalzes zur Verfügung steht. Der pH-Wert der zweiten Komponente soll durch das Puffersalz einen Wert oberhalb von 8 annehmen, bevorzugt liegt der pH-Wert der zweiten Komponente zwischen 8,2 und 9,5.

Es ist weiterhin bevorzugt, daß als Säure zur Einstellung des pH-Werts der ersten Komponente (A) eine Hydroxycarbonsäure mit 2-6 C-Atomen und als Puffersalz in der zweiten Komponente (B) ein Alkalisalz der gleichen Hydroxycarbonsäure mit 2 - 6 C-Atomen enthalten ist.

Als weitere Bestandteile der zweiten Komponente des erfindungsgemäßen Mundspülmittels sind Tenside, Geschmacksstoffe und mundhygienische Wirkstoffe zu nennen.

Als Tenside können anionische, kationische, zwitterionische, ampholytische und nichtionogene oberflächenaktive Substanzen mit einer Wasserlöslichkeit von wenigstens 1 Gew.-% (20°C) in Mengen von 0,1-5 Gew.-%, bevorzugt von 0,1-1 Gew.-%, eingesetzt werden.

Als oberflächenaktive Stoffe sind dabei bevorzugt solche Stoffe zu verstehen, die eine lipophile, lineare Alkyl- oder Acylgruppe mit 10-22 C-Atomen und eine wasserlöslichmachende ionische Gruppe, z.B. eine Sulfat-, Sulfonat-, Phosphat-, Carboxylat- oder z.B. eine Trimethylammoniumgruppe oder eine Acetobetain-Gruppe oder eine nichtionische Polyhydroxyalkyl- oder Polyoxyethylengruppe enthalten. Beispiele für geeignete ionische Tenside sind z.B. Natriumlaurylsulfat, Natrium-lauroylisethionat, Cetyltrimethylammoniumchlorid, Lauryl-trimethylammonium-acetobetain, Lauroylamidopropyl-dimethylammonium-acetobetain. Bevorzugt sind aber nichtionische oberflächenaktive Stoffe enthalten, als Beispiele werden Anlagerungsprodukte von Ethylenoxid an Fettalkohole, an Fettsäuren, an Fettsäuremonoglyceride, an Sorbitanfettsäureester, an Propylenglycolmonofettsäureester oder an Methylglycosid-monofettsäureester genannt. Weitere, bevorzugt geeignete nichtionische Tenside sind Alkyl-(oligo)-glycoside. Alkyl-(oligo)-glycoside, ihre Herstellung und Verwendung als oberflächenaktive Stoffe sind z.B. aus US-A-3,839,318, DE-A-20 36 472, EP-A-77 167 oder WO-A-93/10132 bekannt.

Bezüglich des Glycosidrestes gilt, daß sowohl Monoglycoside (x = 1), bei denen ein Hexoserest glycosidisch an einen Fettalkohol mit 10 bis 16 C-Atomen gebunden ist, als auch oligomere Glycoside mit einem Oligomerisationsgrad x bis 10 geeignet sind. Der Oligomerisationsgrad ist dabei ein statistischer Mittelwert, dem eine für solche technischen Produkte übliche Homologenverteilung zugrunde liegt.

Bevorzugt eignet sich als Alkyl-(oligo)-glycosid ein Alkyl-(oligo)-glucosid der Formel RO(C₆H₁₀O)ₓ-H, in der R eine Alkylgruppe mit 12 bis 14 C-Atomen ist und x einen Mittelwert von 1 bis 4 hat.

Außer den genannten Alkylglucosid-Tensiden können auch andere nichtionische, ampholytische und kationische Tenside enthalten sein. Insbesondere zur Solubilisierung der meist wasserunlöslichen Aromaöle kann ein nichtionogener Lösungsvermittler aus der Gruppe der oberflächenaktiven Verbindungen erforderlich sein. Besonders geeignet für diesen Zweck sind z.B. oxethylierte Fettsäureglyceride, oxethylierte Fettsäuresorbitanpartialester oder Fettsäurepartialester von Glycerin- oder Sorbitan-Oxethylaten. Lösungsvermittler aus der Gruppe der oxethylierten Fettsäureglyceride umfassen vor allem Anlagerungsprodukte von 20 bis 60 Mol Ethylenoxid an Mono- und Diglyceride von linearen Fettsäuren mit 12 bis 18 C-Atomen oder an Triglyceride von Hydroxyfettsäuren wie Oxystearinsäure oder Ricinolsäure. Weitere geeignete Lösungsvermittler sind oxethylierte Fettsäuresorbitanpartialester; das sind bevorzugt Anlagerungsprodukte von 20 bis 60 Mol Ethylenoxid an Sorbitanmonoester und Sorbitandiester von Fettsäuren mit 12 bis 18 C-Atomen. Ebenfalls geeignete Lösungsvermittler sind Fettsäurepartialester von Glycerin- oder Sorbitan-Oxethylaten; das sind bevorzugt Mono- und Diester von C₁₂-C₁₈-Fettsäuren und Anlagerungsprodukten von 20 bis 60 Mol Ethylenoxid an 1 Mol Glycerin oder an 1 Mol Sorbit.

Die erfindungsgemäßen Mundspülmittel enthalten bevorzugt als Lösungsvermittler für gegebenenfalls enthaltene Aromaöle Anlagerungsprodukte von 20 bis 60 Mol Ethylenoxid an gehärtetes oder ungehärtetes Ricinusöl (d.h. an Oxystearinsäure- oder Ricinolsäuretriglycerid), an Glycerin-mono- und/oder -distearat oder an Sorbitanmono- und/oder - distearat.

Als Geschmacksstoffe sind z.B. Süßungsmittel und/oder Aromaöle enthalten. Als Aromaöle kommen alle für Mund- und Zahnpflegemittel gebräuchlichen natürlichen und synthetischen Aromen in Frage. Natürliche Aromen können sowohl in Form der aus den Drogen isolierten etherischen Öle als auch der aus diesen isolierten Einzelkomponenten verwendet werden. Bevorzugt sollte wenigstens ein Aromaöl aus der Gruppe Pfefferminzöl, Krausenminzöl, Anisöl, Sternanisöl, Kümmelöl, Eukalyptusöl, Fenchelöl, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte synthetisch erzeugten Komponenten dieser Öle enthalten sein. Die wichtigsten Komponenten der genannten Öle sind z.B. Menthol, Carvon, Anethol, Cineol, Eugenol, Zimtaldehyd, Caryophyllen, Geraniol, Citronellol, Linalool, Salven, Thymol, Terpinen, Terpinol, Methylchavicol und Methylsalicylat. Weitere geeignete Aromen sind z.B. Menthylacetat, Vanillin, Jonone, Linalylacetat, Rhodinol und Piperiton.

Als Süßungsmittel eignen sich z.B. Saccharin-Natrium, Natrium-cyclamat, Acesulfam-K, Aspartam, Lactose, Maltose oder Fructose.

Schließlich können die erfindungsgemäßen Zweikomponenten-Mundspülmittel mundhygienische und therapeutische Wirkstoffe enthalten, die bevorzugt der zweiten Komponente zugesetzt werden. Solche geeigneten Wirkstoffe sind z.B.
- karieshemmende Fluorverbindungen, z.B. Natriumfluorid, Zinnfluorid, Natriummonofluorophosphat oder Aminfluorid
- Antizahnsteinwirkstoffe, z.B. Organophosphonate, Natrium-pyrophosphat, Natriumtripolyphosphat,
- wundheilende und entzündungshemmende Stoffe wie z.B. Allantoin, Harnstoff, Azulen bzw. Kamillenextrakt,
- antibakterielle, plaquehemmende Stoffe, wie z.B. Chlorhexidin oder Triclosan.

Schließlich können einer oder beiden Komponenten des erfindungsgemäßen Mundspülmittels Trübungsmittel oder Farbstoffe zugesetzt werden.

Die Herstellung der beiden Komponenten kann einfach durch Vermischen der Bestandteile erfolgen. Als Verpackung eignen sich Flaschen, die in einem gemeinsamen Umkarton angeboten werden und mit einer Gebrauchsanweisung versehen sind, aus der sich das Mengenverhältnis entnehmen läßt, in welchem die beiden Komponenten vor der Anwendung gemischt werden.

Es sind aber bereits spezielle Spendebehälter für Zweikomponenten-Mundspülmittel, z.B. aus US 5,392,947 oder aus WO 92/04007 bekannt, die auch für die erfindungsgemäßen Mundspülmittel geeignet sind.

Das erfindungsgemäße Mundspülmittel weist eine unerwartet hohe Reinigungswirkung gegenüber Zahnbelägen und Verfärbungen der Zähne auf, die durch Tee, Kaffee, Nikotin oder färbende Früchte verursacht sind. Diese Reinigungswirkung ist insbesondere stark gesteigert gegenüber einer Wasserstoffperoxid-Lösung von pH = 6 oder gegenüber einer Lösung des Puffersalzes (ohne Wasserstoffperoxid). Diese überraschende Wirkung wird in den folgenden Beispielen näher dargelegt:

### Beispiele

### Zweikomponenten-Mundspülmittel

### 1. Rezepturen

| **Zusammensetzung** **Komponente 1** | **1a** | **1V** |
|---|---|---|
| Wasserstoffperoxid (30 %ig) | 0,67 | 0,67 |
| Zitronensäure-monohydrat | 0,00083 | - |
| Wasser (dest.) | ad 100 | ad 100 |
| pH - Wert | 4,2 | 6,0 |

| **Komponente 2** | **2a** | **2b** | **2V** |
|---|---|---|---|
| Plantaren 1200 UP | 0,1 | 0,1 | 0,1 |
| Cremophor RH60 | 0,2 | 0,2 | 0,2 |
| Aroma | 0,1 | 0,1 | 0,1 |
| Saccharin-Na | 0,03 | 0,03 | 0,03 |
| NaF | 0,045 | 0,045 | 0,045 |
| Sorbit | 1,8 | 1,8 | 1,8 |
| Ethanol | 10,0 | 10,0 | 10,0 |
| Farbstoff | 0,001 | 0,001 | 0,001 |
| Na-Citrat | 5,0 | - | - |
| AHP-Na-Salz | - | 5,0 | - |
| Wasser ad 100 Gew.-% | ad 100 | ad 100 | ad 100 |
| pH-Wert | 8,7 | 8,5 | 7,5 |

### 2. Prüfung der Bleichwirkung

### Prüfmethode

Die Oberfläche von Rinderschneidezähnen wurde konditioniert, unter definierten Bedingungen mit Tee eingefärbt und unter definierten Bedingungen mit dem zu prüfenden 2-Komponenten-Mundwasser behandelt. Die dabei erzielte Aufhellung wurde farbmetrisch untersucht.

### Probenvorbereitung

Rinder-Schneidezähne wurden in Blöcke von 7 x 7 mm geschnitten und die Blöcke mit Wachs so auf Plexiglas-Würfel (1 x 2,5 x 2,5 cm) montiert, daß nur die Schmelzoberfläche frei bleibt. Die Schmelzoberfläche wurde poliert bis sie einheitlich glatt erscheint.

### Probenkonditionierung

Die montierten Zahnblöcke wurden nacheinander in 0,12 N Salzsäure (60 sec.), gesättigte Na₂CO₃-Lösung (120 sec.) und in 1 %ige Phytinsäure-Lösung (60 sec.) eingetaucht. Nach jeder Behandlung wurden die Proben mit entsalztem Wasser gespült und mit saugfähigem Papier trocken getupft. Die Zahnproben wurden dann in das Anschmutzungsgerät eingehängt und 5 Tage lang durch eine Lösung von schwarzem Tee von 20°C bewegt. Die Teelösung wurde durch Extraktion eines 1,5 g Teebeutels mit 300 g kochenden Wassers (10 Min.) zubereitet und zweimal pro Tag erneuert.

### Bleichversuche

Es wurden jeweils 50 ml der Komponente 1 und 50 ml der Komponente 2 (gemäß Tabelle) bei 20°C gemischt und die konditionierten und angeschmutzten Proben für 10 Minuten in die auf 40°C erwärmte Mischung eingelegt, dann herausgenommen, mit entsalztem Wasser gespült und trocken getupft und dann farbmetrisch gemessen.

Es wurde die Differenz des Grauwertes [ΔL der Farbmeßmethode nach CIELAB] zwischen der unbehandelten und einer gebleichten Probe bestimmt.

Dabei wurde das folgende Ergebnis erhalten (Tabelle):

| **Beispiel** | **V1** | **V2** | **V3** | **1** | **2** |
|---|---|---|---|---|---|
| Mundspülmittel aus Komponente 1 | 1V | - | - | 1a | 1a |
| und Komponente 2 | - | 2V | 2a | 2a | 2b |
| pH-Wert | 6,0 | 7,5 | 8,7 | 7,1 | 7,2 |
| ΔL | 1,5 | 0,7 | 0,9 | 4,9 | 4,5 |

## Patentansprüche

1. Mundspülmittel, bestehend aus zwei getrennt verpackten Komponenten, die in einer Verkaufseinheit zusammengefasst sind und vor der Anwendung miteinander gemischt werden, **dadurch gekennzeichnet, dass**
- die erste Komponente (A) eine wässrige Lösung von unter 0,5 Gew.-% Wasserstoffperoxid und eine Säure zur Einstellung eines pH-Werts unter 4,5 und
- die zweite Komponente (B) eine wäßrige oder wäßrig-alkoholische Lösung mit einem oder mehreren Stoffen aus der Gruppe Tenside, Geschmacksstoffe und mundhygienische Wirkstoffe und 1-10 Gew.-% eines Puffersalzes einer nichtflüchtigen Säure umfaßt und einen pH-Wert oberhalb 8 aufweist und
- die Mischung der beiden Komponenten eine Lösung mit einem Wasserstoffperoxidgehalt von mindestens 0,005 Gew.-% und mit einem pH-Wert von 7 oder höher ergibt.

2. Mundspülmittel nach Anspruch 1, **dadurch gekennzeichnet, dass** als Puffersalze in der zweiten Komponente (B) die Salze von komplexbildenden Säuren aus der Gruppe der Hydroxy- oder Aminopolycarbonsäuren, der Organophosphonsäuren oder von kondensierten Oligophosphorsäuren enthalten sind.

3. Mundspülmittel nach einem der Ansprüche 1 - 2, **dadurch gekennzeichnet, dass** als Puffersalz Trinatriumcitrat in einer Menge von 3-6 Gew.-% in der zweiten Komponente (B) enthalten ist.

4. Mundspülmittel nach einem der Ansprüche 1 - 3, **dadurch gekennzeichnet, dass** als Säure zur Einstellung des pH-Werts in der ersten Komponente (A) eine Hydroxycarbonsäure mit 2 - 6 C-Atomen und als Puffersalz in der zweiten Komponente (B) ein Alkalisalz der gleichen Hydroxycarbonsäure mit 2-6 C-Atomen enthalten ist.

## Claims

1. A mouthwash consisting of two separately packed components which are combined into a single retail unit and which are mixed together before use, **characterized in that**
- the first component (A) contains an aqueous solution of less than 0.5% by weight hydrogen peroide and an acid for establishing a pH below 4.5. and
- the second component (B) contains an aqueous or aqueous/alcohlic solution of one or more substances from the group of surfactants, flavours and oral hygiene components and 1 to 10% by weight of a buffer salt of a nonvolatile acid and has a pH above 8 and
- mixing of the two components gives a solution with a hydrogen peroide content of at least 0.005% by weight and a pH of 7 or higher.

2. A mouthwash as claimed in claim 1, **characterized in that** the buffer salts present in the second component (B) are the salts of complexing acids from the group of hydroxy- or aminopolycarboxylic acids, organophosphonic acid or condensed oligophosphoric acids.

3. A mouthwash as claimed in claim 1 or 2, **characterized in that** trisodium citrate in a quantity of 3 to 6% by weight is present as the buffer salt in the second component (B).

4. A mouthwash as claimed in any of claims 1 to 3, **characterized in that** a C₂₋₆ hydroxycarboxylic acid is present as the acid for pH adjustment in the first component (A) and an alkali metal salt of the same C₂₋₆ hydroxycarboxylic acid is present as the buffer salt in the second component (B).

## Revendications

1. Bain de bouche constitué de deux composants conditionnés séparément, qui sont rassemblés dans une unité de vente et que l'on mélange entre eux avant l'emploi, **caractérisé en ce que**
- le premier composant (A) est une solution aqueuse de moins de 0,5% en poids de peroxyde d'hydrogène et d'un acide pour établir un pH inférieur à 4,5 et
- le second composant (B) comprend une solution aqueuse ou alcoolique aqueuse avec un ou plusieurs corps du groupe des agents tensioactifs, des rehausseurs de goût et des substances actives hygiéniques pour la bouche et de 1 à 10% en poids d'un sel tampon d'un acide non volatil et présente un pH supérieur à 8 et
- le mélange des deux composants donne une solution ayant une teneur en peroxyde d'hydrogène d'au moins 0,005% en poids et ayant un pH égal ou supérieur à 7.

2. Bain de bouche selon la revendication 1, **caractérisé en ce qu'**on trouve comme sels tampon dans le second composant (B) les sels d'acides formateurs de complexes du groupe des acides hydroxy- ou aminopolycarboxyliques, des acides organo-phosphoriques ou des acides oligophosphoriques condensés.

3. Bain de bouche selon l'une des revendications 1 ou 2, **caractérisé en ce qu'**on trouve dans le second composant (B) comme sel tampon le citrate trisodique en une quantité de 3 à 6% en poids.

4. Bain de bouche selon l'une des revendications 1 à 3, **caractérisé en ce qu'**on trouve comme acide pour la régulation du pH dans le premier composant (A) un acide hydroxycarboxylique comportant de 2 à 6 atomes de carbone et comme sel tampon dans le second composant (B) un sel de métal alcalin du même acide hydroxycarboxylique comportant de 2 à 6 atomes de carbone.
